# EUROPÄISCHE PATENTSCHRIFT

(11) **EP 3 687 366 B1**
(45) Veröffentlichungstag und Bekanntmachung des Hinweises auf die Patenterteilung: **01.11.2023**
(21) Anmeldenummer: 18765633.5
(22) Anmeldetag: 06.09.2018
(51) Int. Cl.: A61B 1/00, A61B 1/12

(54) **VERFAHREN ZUM AUFBEREITEN EINES WENIGSTENS EINEN KANAL AUFWEISENDEN ENDOSKOPS IN EINEM AUFBEREITUNGSGERÄT**
METHOD FOR PREPARING AN ENDOSCOPE, WHICH HAS AT LEAST ONE CHANNEL, IN A PREPARATION DEVICE
PROCÉDÉ POUR PRÉPARER UN ENDOSCOPE PRÉSENTANT AU MOINS UN CANAL DANS UN APPAREIL DE PRÉPARATION

(30) Priorität: 27.09.2017 DE 102017122434
(43) Veröffentlichungstag der Anmeldung: 05.08.2020
(73) Patentinhaber: Olympus Winter & Ibe GmbH, 22045 Hamburg (DE)
(72) Erfinder: CARLSON, Torben, 22047 Hamburg (DE); VELIC, Ramiza, 22177 Hamburg (DE)
(74) Vertreter: Noack, Andreas
(86) Internationale Anmeldenummer: PCT/EP2018/074023
(87) Internationale Veröffentlichungsnummer: WO 2019/063261

(56) Entgegenhaltungen:
- EP-A1- 2 060 276
- JP-A- 2006 230 709
- US-A1- 2009 220 377
- US-A1- 2015 320 303

## Beschreibung

Die Erfindung betrifft ein Verfahren zum Aufbereiten eines wenigstens einen Kanal aufweisenden Endoskops in einem Aufbereitungsgerät, mit den Schritten: Einbringen des Endoskops in das Aufbereitungsgerät, Verbinden von wenigstens einem Kanal des Endoskops mit einem Spülsystem des Aufbereitungsgeräts, Durchspülen des wenigstens einen Kanals des Endoskops mit einer Spülflüssigkeit, wobei eine Durchflussrate der Spülflüssigkeit durch den wenigstens einen Kanal gemessen wird, um eine vollständige oder teilweise Blockierung des wenigstens einen Kanals oder eine unvollständige Verbindung des wenigstens einen Kanals mit dem Spülsystem zu erkennen.

Endoskope werden seitlängerer Zeit insbesondere in der Medizin eingesetzt, um schwer zugängliche Bereiche im Körper eines menschlichen oder tierischen Patienten zu untersuchen und/oder zu behandeln. Dazu weisen Endoskope zumeist einen langgestreckten Schaft auf, an dessen distalen Ende sich ein Objektiv befindet, durch welches ein interessierender Bereich im Körper des Patienten betrachtet werden kann. Am proximalen Ende des Schaftes befindet sich zumeist ein Hauptkörper, welcher zur Handhabung des Endoskops dient.

Endoskope weisen oftmals innere Kanäle auf, durch welche Fluide an das distale Ende des Endoskops gefördert werden können, um dort abgegeben zu werden. Alternativ können durch entsprechende Kanäle auch Fluide am distalen Ende des Endoskops aufgenommen und in Richtung des Hauptkörpers abgesaugt werden. Weiterhin können Behandlungsinstrumente durch entsprechende Kanäle des Endoskops zum distalen Ende vorgeschoben werden, um einen Eingriff an dem interessierenden Bereich des Körpers vorzunehmen.

Endoskope sind in der Herstellung sehr aufwendig und daher teuer. Um Kosten zu sparen werden Endoskope daher mehrfach verwendet. Dies erfordert eine gründliche Aufbereitung der Endoskope zwischen zwei Anwendungen, um die Übertragung von Krankheiten zu verhindern.

Zur Aufbereitung eines Endoskops werden in der Regel nach einer manuellen Vorreinigung die Kanäle des Endoskops in einem Aufbereitungsgerät nacheinander mit unterschiedlichen Reinigungs- und/oder Desinfektionsflüssigkeiten durchspült. Dazu werden die Kanäle des Endoskops mit einem Spülsystem des Aufbereitungsgeräts verbunden.

Für eine wirksame Aufbereitung des Endoskops ist es unbedingt erforderlich, dass die Kanäle des Endoskops durchgängig sind und korrekt mit dem Spülsystem verbunden sind.

Ist ein Kanal ganz oder teilweise blockiert, beispielsweise durch Gewebereste oder koagulierte Körperflüssigkeiten, so können die Spülflüssigkeiten nicht alle Bereiche des entsprechenden Kanals erreichen. Ist ein Kanal nicht korrekt mit dem Spülsystem verbunden, so kann die Spülflüssigkeit ebenfalls nicht alle Bereiche des Kanals korrekt erreichen. In beiden Fällen besteht die Gefahr, dass Krankheitserreger in dem Kanal verbleiben und somit ein Infektionsrisiko für einen nachfolgend zu behandelnden Patienten darstellen.

Es ist daher vorgeschrieben, dass das Aufbereitungsgerät die Durchlässigkeit und die korrekte Verbindung der Kanäle eines aufzubereitenden Endoskops mit dem Spülsystem selbsttätig prüft. Dazu weisen Aufbereitungsgeräte eine Durchflusskontrolleinheit auf, welche eine Durchflussrate der Spülflüssigkeit durch den wenigstens einen Kanal misst, um eine vollständige oder teilweise Blockierung des wenigstens einen Kanals oder eine unvollständige Verbindung des wenigstens einen Kanals mit dem Spülsystem zu erkennen.

Solche beispielsweise aus US2015/320303 A1 oder DE10352198 A1 bekannte Durchflusskontrolleinheiten arbeiten in der Regel so, dass die Durchflussrate der Spülflüssigkeit durch einen Kanal gemessen wird, wenn sich in dem Kanal ein stationärer Strömungszustand eingestellt hat. Dann werden zumeist mehrere einzelne Messwerte der Durchflussrate aufgenommen und gemittelt, um eine mittlere Durchflussrate zu bestimmen. Diese mittlere Durchflussrate wird dann mit zulässigen Wertebereichen verglichen. Liegt die mittlere Durchflussrate nicht in einem zulässigen Wertebereich, so wird der Aufbereitungsvorgang abgebrochen und der Benutzer aufgefordert, dass Endoskop zu reinigen bzw. korrekt anzuschließen.

Während das beschriebene Arbeitsverfahren der Durchflusskontrolleinheit zuverlässig und sicher funktioniert, weist es doch gewisse Nachteile auf. Insbesondere stellt sich ein stationärer Strömungszustand in den Endoskopkanälen nicht sofort nach Beginn eines Spüldurchgangs ein, sondern abhängig vom Aufbau des Aufbereitungsgeräts erst nach mehreren Minuten. Muss dann der Aufbereitungsprozess unterbrochen werden, so ist die verlorene Vorlaufzeit ärgerlich, gerade wenn beispielsweise in einem Krankenhaus eine große Anzahl von Endoskopen in kurzer Zeit aufbereitet werden muss.

Es besteht daher die Aufgabe der Erfindung daran, ein Verfahren zum Aufbereiten eines wenigstens einen Kanal aufweisenden Endoskops in einem Aufbereitungsgerät bereitzustellen, welches, insbesondere hinsichtlich der Erkennung von blockierten oder nicht korrekt angeschlossenen Kanälen, verbessert ist.

Diese Aufgabe wird gemäß der Erfindung gelöst durch ein Verfahren zum Aufbereiten eines wenigstens einen Kanal aufweisenden Endoskops in einem Aufbereitungsgerät, mit den Schritten: Einbringen des Endoskops in das Aufbereitungsgerät, Verbinden von wenigstens einem Kanal des Endoskops mit einem Spülsystem des Aufbereitungsgeräts, Durchspülen des wenigstens einen Kanals des Endoskops mit einer Spülflüssigkeit, wobei eine Durchflussrate der Spülflüssigkeit durch den wenigstens einen Kanal gemessen wird, um eine vollständige oder teilweise Blockierung des wenigstens einen Kanals oder eine unvollständige Verbindung des wenigstens einen Kanals mit dem Spülsystem zu erkennen, welches dadurch weitergebildet ist, dass die Durchflussrate über einen vorbestimmten Zeitraum mehrfach gemessen wird, und dass die Änderung der Durchflussrate über die Zeit verwendet wird, um eine Blockierung des wenigstens einen Kanals oder eine unvollständige Verbindung des wenigstens einen Kanals mit dem Spülsystem zu erkennen.

Die Erfindung beruht auf der Erkenntnis, dass aus dem zeitlichen Verlauf der Durchflussrate einer Spülflüssigkeit durch den Kanal eines Endoskops bereits hinreichend genaue Rückschlüsse auf die Durchlässigkeit oder den korrekten Anschluss des Kanals gezogen werden können, bevor sich in dem Kanal ein stationärer Strömungszustand eingestellt hat. Es ist somit nicht mehr erforderlich, erst den stationären Strömungszustand abzuwarten, so dass eine etwaige Blockierung und/oder ein unvollständiger Anschluss des Kanals deutlich früher erkannt werden können.

In einer möglichen Weiterbildung des Verfahren gemäß der Erfindung werden zum Aufbereiten des Endoskops mehrere Spüldurchgänge durchgeführt, und die Durchflussrate wird über mehrere vorbestimmte Zeiträume, welche mit jeweils einem der Spüldurchgänge zusammenfallen, gemessen, und die Änderung der Durchflussrate über die Zeit wird für jeden vorbestimmten Zeitraum separat ausgewertet.

Durch die mehrfache Auswertung des zeitlichen Verlaufs der Durchflussrate in mehreren Spülgängen können auch Störungen bei der Durchspülung der Kanäle eines Endoskops erkannt werden, die erst im Verlauf der Aufbereitung auftreten. So können beispielsweise Ablagerungen aus einem Kanalabschnitt mit einem großen Querschnitt während der Aufbereitung abgelöst und in einen engeren Kanalabschnitt gespült werden, wo diese Ablagerungen dann eine Blockierung hervorrufen. Ebenso könnte sich eine nicht vollständig gesicherte Verbindung eines Kanals mit dem Spülsystem im Laufe des Aufbereitungsprozesses lösen. Die beiden beschriebenen Fehlerfälle würden durch ein entsprechend ausgestaltetes Aufbereitungsverfahren erkannt.

In einer möglichen Ausgestaltung eines erfindungsgemäßen Verfahrens ist das Spülsystem ein Umlauf-Spülsystem, welches zu Beginn eines Spüldurchgangs mit Spülflüssigkeit befüllt wird, und nach Beendigung eines Spüldurchgangs von der Spülflüssigkeit entleert wird, und der vorbestimmte Zeitraum ist so gewählt, dass er zumindest teilweise mit dem Befüllen des Spülsystems zusammenfällt.

Überraschenderweise hat sich herausgestellt, dass die zeitliche Änderung der Durchflussrate der Spülflüssigkeit durch die Kanäle eines Endoskops während der Befüllung des Spülsystems mit Spülflüssigkeit hinreichend reproduzierbar ist, um Rückschlüsse auf die Durchlässigkeit oder die korrekte Verbindung der Kanäle mit dem Spülsystem zu erlauben. Ein Fehlerfall kann somit erkannt werden, sobald mit der Befüllung des Spülsystems begonnen wird, wodurch der Zeitverlust im Fehlerfall deutlich reduziert werden kann.

In einer vorteilhaften Variante eines erfindungsgemäßen Verfahrens werden für die Bestimmung der Änderung der Durchflussrate über die Zeit wenigstens 3, bevorzugt wenigstens 5, besonders bevorzugt wenigstens 7 Messwerte für die Durchflussrate ausgewertet. Dabei kann ein vorbestimmter Zeitraum zwischen 6 Sekunden und 30 Sekunden, bevorzugt zwischen 8 Sekunden und 20 Sekunden, besonders bevorzugt zwischen 10 Sekunden und 15 Sekunden betragen.

Die Bestimmung der Änderung der Durchflussrate über die Zeit kann mittels linearer Regression erfolgen.

Zulässige Werte für die Änderung der Durchflussrate über die Zeit können zu Beginn des Aufbereitungsprozesses aus einer Datenbank gelesen werden. Dazu kann in einer möglichen Ausführungsform der Erfindung zu Beginn des Aufbereitungsprozesses ein Typ des aufzubereitenden Endoskops bestimmt werden, und dann die für den entsprechenden Endoskoptyp zulässigen Werte für die Änderung der Durchflussrate über die Zeit aus der Datenbank gelesen werden.

Nachfolgend wird die Erfindung anhand einiger beispielhafter Zeichnungen näher erläutert. Es zeigen:
Fig. 1: Den prinzipiellen Aufbau eines Aufbereitungsgeräts für Endoskope,
Fig. 2: den Aufbau einer Durchflusskontrolleinheit,
Fig. 3a,b: den möglichen Ablauf eines Aufbereitungsprozesses,

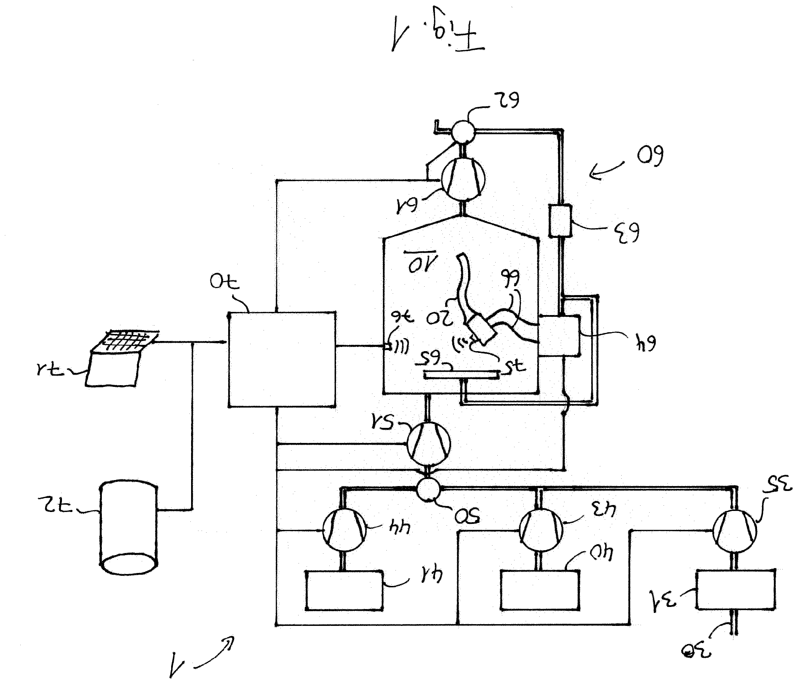

in Figur 1 ist der prinzipielle Aufbau eines Aufbereitungsgeräts 1 für Endoskope dargestellt.

Das Aufbereitungsgerät 1 umfasst einen Aufbereitungsraum 10, in welchem ein aufzubereitendes Endoskop 20 angeordnet werden kann. Dazu weist der Aufbereitungsraum 10 eine nicht dargestellte Tür auf. Zur Aufbereitung des Endoskops 20 wird dieses in den Aufbereitungsraum 10 eingebracht und in diesem mit Spülflüssigkeit um- und durchspült.

Zur Bereitstellung der Spülflüssigkeit weist das Aufbereitungsgerät 1 einen Wasserzulauf 30 auf, welcher beispielsweise direkt an ein Trinkwasserversorgungsnetz angeschlossen sein kann. In dem Wasserzulauf 30 kann eine Wasseraufbereitungseinheit 31 vorgesehen sein, welches beispielsweise eine Druckreduzierung, Enthärtung, und/oder eine Entmineralisierung des einlaufenden Wassers bewirken kann. Dabei hängt die Art der Wasseraufbereitungseinheit 31 stark von den Eigenschaften des für den Wasserzulauf 30 bereitgestellten Wassers ab. An die Wasseraufbereitungseinheit 31 schließt sich eine Dosierpumpe 35 an.

Das Aufbereitungsgerät 1 umfasst weiterhin Vorratsbehälter 40,41, in welchen Reinigungs- und/oder Desinfektionsmittel vorgehalten werden. Die Vorratsbehälter 40,41 sind mit weiteren Dosierpumpen 43,44 verbunden. Anstelle der gezeigten zwei Vorratsbehälter können auch 3 oder mehr Vorratsbehälter vorgesehen sein.

Hinter den Dosierpumpen 35, 43,44 ist eine Mischkammer 50 vorgesehen, welche wiederum über eine Förderpumpe 51 mit dem Aufbereitungsraum 10 verbunden ist.

Während der Aufbereitung des Endoskops 20 werden nacheinander verschiedene Spülflüssigkeiten angemischt, indem die Dosierpumpen 35,43,44 die jeweils benötigten Mengen an Wasser, Reinigungsmittel, und/oder Desinfektionsmittel in die Mischkammer 50 fördern. Aus der Mischkammer 50 wird die fertig gemischte Spülflüssigkeit dann durch die Förderpumpe 51 in den Aufbereitungsraum 10 gefördert.

In dem Aufbereitungsraum 10 ist das Endoskop 20 mit einem Spülsystem verbunden, welches im vorliegenden Beispiel als Umlauf-Spülsystem 60 ausgeführt ist. Das Umlauf-Spülsystem 60 umfasst eine Umwälzpumpe 61, ein Ablaufventil 62, eine Heizung 63, eine Durchflusskontrolleinheit 64 sowie einen Sprüharm 65. Die Durchflusskontrolleinheit 64 ist über Schläuche 66 mit Kanälen des Endoskops 20 verbunden.

Während der Aufbereitung des Endoskops 20 wird die Spülflüssigkeit durch die Umwälzpumpe 61 aus dem Aufbereitungsraum 10 in die Heizung gefördert, wo sie auf die für den jeweiligen Aufbereitungsschritt vorgegebene Temperatur erwärmt wird. Dann wird ein Teil der Spülflüssigkeit zu dem Sprüharm 65 gefördert, welcher die Spülflüssigkeit in dem Aufbereitungsraum 10 verteilt, so dass das Endoskop 10 von außen mit der Spülflüssigkeit benetzt wird. Dazu ist der Sprüharm als passiver Dreharm ausgeführt, welcher lediglich durch den Rückstoß der abgegebenen Spülflüssigkeit bewegt wird.

Ein anderer Teil der Spülflüssigkeit wird zu der Durchflusskontrolleinheit 64 gefördert, hier wird die Spülflüssigkeit auf mehrere Anschlüsse verteilt, welche über die Schläuche 66 mit den Kanälen des Endskops 20 verbunden sind.

In Figur 1 ist der Übersichtlichkeit halber nur ein Endoskop 20 dargestellt, welches mit zwei Ausgängen der Durchflusskontrolleinheit 64 verbunden ist. In den meisten Aufbereitungsgeräten können tatsächlich zwei oder mehr Endoskope gleichzeitig aufbereitet werden, wobei die Endoskope jeweils mit drei oder mehr Ausgängen der Durchflusskontrolleinheit verbunden sein können.

Nach dem Durchlaufen des Sprüharms 65 oder der Kanäle des Endoskops 20 gelangt die Spülflüssigkeit wieder in den Aufbereitungsraum 10 und wird durch die Umwälzpumpe 61 erneut in Richtung der Heizung 63 gefördert.

Am Ende eines jeden Spülgangs wird das Ablaufventil 62 geöffnet, um die verbrauchte Spülflüssigkeit abzufördern.

Die Funktion der Pumpen 35,43,44,51,61, der Heizung sowie des Ablassventils 62 werden von einer Steuerung 70 kontrolliert, die mit einer Benutzerschnittstelle 71, hier als tragbarer Computer dargestellt, und mit einer Datenbank 72 verbunden ist.

Der genaue Ablauf eines Aufbereitungsprozesses kann abhängig von der Art des aufzubereitenden Endoskops variieren. Dazu sind in der Datenbank 72 verschiedene Aufbereitungsprogramme hinterlegt. Ein Benutzer des Aufbereitungsgeräts 1 kann über die Benutzerschnittstelle 71 eines dieser Aufbereitungsprogramme auswählen.

Alternativ kann die Steuerung 70 eigenständig die Art des aufzubereitenden Endoskops 20 erkennen und ein geeignetes Aufbereitungsprogramm auswählen. Dazu kann das Endoskop 20 mit einem Datenträger 75 ausgerüstet sein, welcher mittels eines Lesegeräts 76 durch die Steuerung 70 ausgelesen werden kann. Bei dem Datenträger 75 kann es sich beispielsweise um einen RFID-Chip handeln, entsprechend kann das Lesegerät 76 ein RFID-Lesegerät sein oder umfassen.

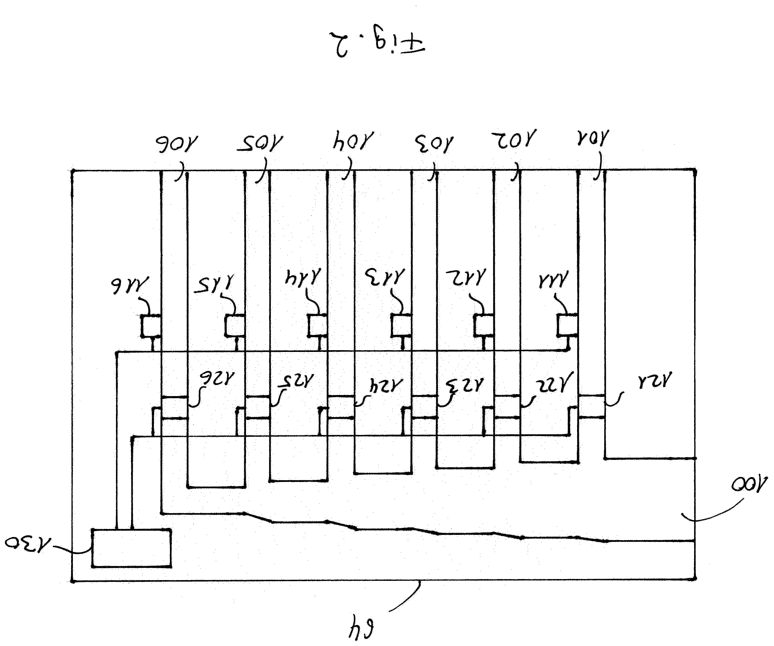

In Figur 2 ist der innere Aufbau der Durchflusskontrolleinheit 64 genauer dargestellt. Die Durchflusskontrolleinheit weist einen Eingangskanal 100 auf, welcher sich im dargestellten Beispiel in 6 Ausgangskanäle 101,102,103,104,105,106 verzweigt. Jedem der Ausgangskanäle 101,102,103,104,105,106 ist ein Strömungssensor 111,112,113,114,115,116 zugeordnet, um die jeweilige Durchflussrate in den Ausgangskanälen 101,102,103,104,105,106 zu messen. Weiterhin kann in jedem der Ausgangskanäle 101,102,103,104,105,016 ein Absperrventil 121,122,123,124,125,126 angeordnet sein, um den jeweiligen Ausgangskanal zu schließen, beispielsweise wenn an dem jeweiligen Ausgangskanal kein Endoskop angeschlossen ist.

Die Durchflusskontrolleinheit kann eine eigene Steuerungseinheit 130 aufweisen, um mit den Absperrventilen 121,122,123,124,125,126 und den Strömungssensoren 111,112,113,114,115,116 zu kommunizieren. Die Steuerungseinheit 130 kann mit der Steuerung 70 verbunden sein. Alternativ können die Absperrventile 121,122,123,124,125,126 und die Strömungssensoren 111,112,113,114,115,116 über ein Bussystem direkt mit der Steuerung 70 verbunden sein. Als Bussystem kann beispielsweise ein CAN-Bussystem (Controller Area Network) sein.

Die Strömungssensoren 111,112,113,114,115,116 können unterschiedliche Funktionsprinzipien aufweisen. Beispielsweise können die Strömungssensoren 111,112,113,114,115,116 als magnetisch-induktive Strömungssensoren ausgeführt sein. Bei diesen Sensoren werden Ionen in der Flüssigkeit durch ein äußeres Magnetfeld abgelenkt, wodurch zwischen Messelektroden an gegenüberliegenden Wänden einer Leitung eine elektrische Spannung entsteht, welche proportional zu dem Massendurchfluss der Flüssigkeit ist. Für eine magnetisch-induktive Durchflussmessung muss die zu messende Flüssigkeit eine ausreichende elektrische Leitfähigkeit aufweisen, die mindestens bei 20µS/cm liegt. Andere anwendbare Messprinzipien sind beispielsweise die Wirbelfrequenz-Durchflussmessung, Ultraschall-Durchflussmessung oder Coriolis-Durchflussmessung.

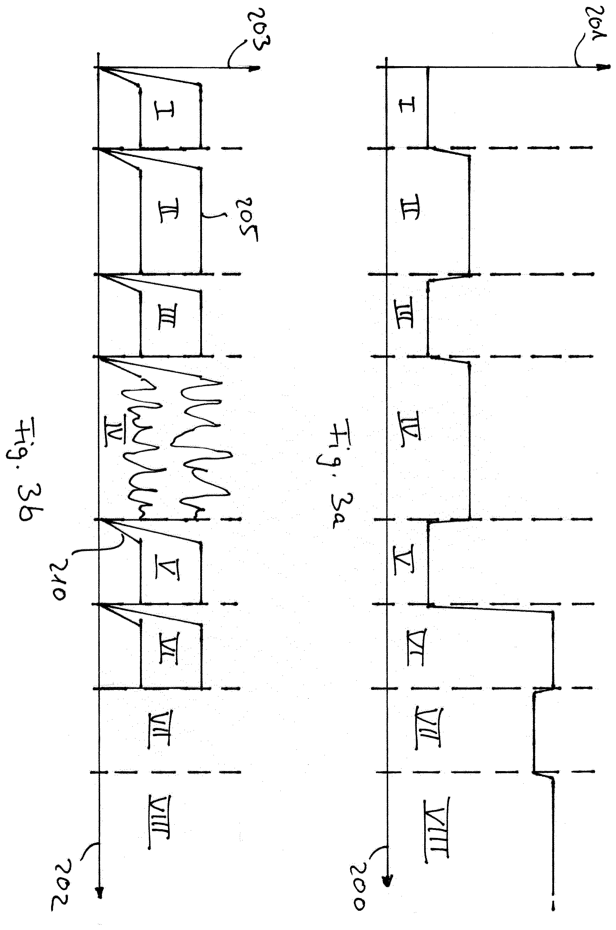

In den Figuren 3a,3b ist ein möglicher Ablauf eines Aufbereitungsverfahrens dargestellt. Dabei zeigt die Figur 3a die Prozessdauer (horizontale Achse 200) und Prozesstemperatur (vertikale Achse 201) von acht Prozessschritten I, II, III, IV, V, VI, VII, VIII.

Im Prozessschritt I erfolgt eine Vorspülung mit kaltem Wasser (20°C-30°C), die mindestens 0,5 Minuten dauern soll. In herkömmlichen Aufbereitungsverfahren wird in diesem Prozessschritt auch die Durchflussmessung durchgeführt, hierzu muss aber der Prozessschritt deutlich ausgedehnt werden, da sich erst nach einigen Minuten ein ausreichend stationärer Strömungszustand in dem Spülsystem eingestellt hat.

Im Prozessschritt II erfolgt die Reinigung zum Entfernen von Schmutz und Geweberesten. Dazu wird das Endoskop für 3 Minuten mit einer Mischung aus Wasser und Reinigungsmittel durchspült. Die Prozesstemperatur in diesem Prozessschritt beträgt 35°C, die Prozessdauer beginnt erst zu laufen, wenn diese Temperatur erreicht ist.

Im Prozessschritt III werden Reste der Reinigungslösung durch Spülen mit kaltem Wasser entfernt. Der Prozessschritt dauert etwa 0,5 Minuten.

Im Prozessschritt IV wird das Endoskop desinfiziert, dazu wird eine Mischung aus Wasser, einem Desinfektionsmittel, und ggf. einem Aktivator für das Desinfektionsmittel gespült. Die Prozesstemperatur beträgt etwa 35°C, die Prozessdauer beträgt etwa 5 Minuten.

Im Prozessschritt V werden Reste der Desinfektionslösung durch Spülen mit kaltem Wasser entfernt. Der Prozessschritt dauert etwa eine Minute.

Im Prozessschritt VI folgt ebenfalls für etwa eine Minute eine abschließende Spülung mit Wasser bei etwa 57°C.

Anschließend wird das Endoskop im Prozessschritt VII für ca. 2 Minuten abgekühlt und dann in Prozessschritt VIII für ca. 15 Minuten bei etwa 57°C getrocknet.

In Figur 3b ist für den gleichen Aufbereitungsprozess die gemessene Durchflussrate der Spülflüssigkeiten in zwei Kanälen des Endoskops 20 dargestellt. Dabei zeigt die horizontale Achse 202 wiederum die Prozessdauer, während auf der vertikalen Achse 203 die Durchflussrate dargestellt ist.

Die Linie 205 zeigt den Verlauf der gemessenen Durchflussrate der Spülflüssigkeit in einem Kanal des Endoskops 20 mit relativ großem Durchmesser, beispielsweise einem Biopsiekanal, während die Linie 210 den Verlauf der gemessenen Durchflussrate in einem Kanal des Endoskops 20 mit relativ geringem Durchmesser zeigt, beispielsweise einem Spülkanal.

Es ist erkennbar, dass nur in den Prozessschritten I - VI überhaupt Durchflussraten gemessen werden, da in den Prozessschritten VII und VIII keine Flüssigkeit durch das Endoskop 20 gefördert wird. In den Prozessschritten I - VI werden jeweils etwa 10 I bis 11I Spülflüssigkeit in dem Spülsystem 60 umgewälzt.

Im Prozessschritt IV schwanken die gemessene Durchflussrate stark. Dies ist dadurch zu erklären, dass das verwendete Desinfektionsmittel eine Schaumbildung bewirkt, welche die Durchflussmessung mittels der Strömungssensoren 111,112,113,114,115,116 beeinträchtigt.

Es ist weiter erkennbar, dass zu Beginn eines jeden Prozessschritts die gemessene Durchflussrate linear ansteigt, bis ein Plateau erreicht ist. Dieser lineare Anstieg ist auch in Prozessschritt IV erkennbar, bei dem ansonsten kein klares Plateau vorhanden ist.

Der lineare Anstieg der Durchflussrate zeigt die Phase des Prozessschritts an, in welchem die Spülflüssigkeit durch die Förderpumpe 51 in den Aufbereitungsraum 10 zugeführt wird. Die Steigung der Durchflussrate über die Zeit ist in dieser Phase im Wesentlichen von der Durchlässigkeit des jeweiligen Kanals abhängig und somit gut geeignet, eine Blockierung oder unvollständige Verbindung eines Kanals zu erkennen.

Um die Steigung zu bestimmen, werden durch die Steuerung 70 mittels der Strömungssensoren 111,112,113,114,115,116 in dieser Phase mehrere Messungen der Durchflussrate durchgeführt, und aus den gewonnenen Messwerten mittel linearer Regression die Steigung bestimmt. Es können beispielsweise in einem Zeitfenster zwischen 45 Sekunden und 60 Sekunden nach Beginn des jeweiligen Prozessschrittes zwischen 5 und 10 Messwerte, beispielsweise 7 Messwerte, gewonnen werden.

Die Steuerung vergleicht die ermittelten Steigungen dann mit zulässigen Werten, welche zuvor aus der Datenbank 72 geladen wurden. Liegt ein Steigungswert außerhalb des zulässigen Bereichs, so deutet dies darauf hin, dass der entsprechende Kanal des Endoskops 20 blockiert oder nicht korrekt angeschlossen ist. In diesem Fall stoppt die Steuerung 70 den Aufbereitungsprozess und fordert den Benutzer über die Benutzerschnittstelle 71 auf, den Fehler zu beheben.

Dadurch, dass die Fehlererkennung kurz nach dem Beginn der jeweiligen Prozessschritte erfolgt, geht hierbei nur wenig Zeit verloren. Gleichzeitig kann das beschriebene Verfahren auch Fehler erkennen, die erst im Laufe des Aufbereitungsprozesses entstehen, beispielsweise durch Einspülen einer Schmutzablagerung in einen engen Kanalabschnitt, oder durch Abrutschen eines Schlauchs von einem Anschluss.

## Patentansprüche

1. Verfahren zum Aufbereiten eines wenigstens einen Kanal aufweisenden Endoskops (20) in einem Aufbereitungsgerät (1), mit den Schritten:
- Einbringen des Endoskops (20) in das Aufbereitungsgerät (1),
- Verbinden von wenigstens einem Kanal des Endoskops (20) mit einem Spülsystem des Aufbereitungsgeräts (1),
- Durchspülen des wenigstens einen Kanals des Endoskops (1) mit einer Spülflüssigkeit,
wobei eine Durchflussrate der Spülflüssigkeit durch den wenigstens einen Kanal gemessen wird, um eine vollständige oder teilweise Blockierung des wenigstens einen Kanals oder eine unvollständige Verbindung des wenigstens einen Kanals mit dem Spülsystem zu erkennen, und
die Durchflussrate über einen vorbestimmten Zeitraum mehrfach gemessen wird,
**dadurch gekennzeichnet, dass** die Änderung der Durchflussrate über die Zeit verwendet wird, um eine Blockierung des wenigstens einen Kanals oder eine unvollständige Verbindung des wenigstens einen Kanals mit dem Spülsystem zu erkennen.

2. Verfahren nach Anspruch 1, wobei zum Aufbereiten des Endoskops (20) mehrere Spüldurchgänge durchgeführt werden, **dadurch gekennzeichnet, dass** die Durchflussrate über mehrere vorbestimmte Zeiträume, welche mit jeweils einem der Spüldurchgänge zusammenfallen, gemessen wird, und dass die Änderung der Durchflussrate über die Zeit für jeden vorbestimmten Zeitraum separat ausgewertet wird.

3. Verfahren nach Anspruch 1 oder 2, wobei das Spülsystem ein Umlauf-Spülsystem (60) ist, welches zu Beginn eines Spüldurchgangs mit Spülflüssigkeit befüllt wird, und nach Beendigung eines Spüldurchgangs von der Spülflüssigkeit entleert wird, **dadurch gekennzeichnet, dass** der vorbestimmte Zeitraum so gewählt ist, dass er zumindest teilweise mit dem Befüllen des Spülsystems zusammenfällt.

4. Verfahren nach einem der Ansprüche 1 bis 3, **dadurch gekennzeichnet, dass** für die Bestimmung der Änderung der Durchflussrate über der Zeit wenigstens 3, bevorzugt wenigstens 5, besonders bevorzugt wenigstens 7 Messwerte für die Durchflussrate ausgewertet werden.

5. Verfahren nach einem der Ansprüche 1 bis 4, **dadurch gekennzeichnet, dass** ein vorbestimmter Zeitraum zwischen 6 Sekunden und 30 Sekunden, bevorzugt zwischen 8 Sekunden und 20 Sekunden, besonders bevorzugt zwischen 10 Sekunden und 15 Sekunden beträgt.

6. Verfahren nach einem der Ansprüche 1 bis 5, **dadurch gekennzeichnet, dass** die Bestimmung der Änderung der Durchflussrate über die Zeit mittels linearer Regression erfolgt.

7. Verfahren nach einem der vorangehenden Ansprüche, **dadurch gekennzeichnet, dass** zu Beginn des Aufbereitungsprozesses zulässige Werte für die Änderung der Durchflussrate über die Zeit aus einer Datenbank (72) gelesen werden.

8. Verfahren nach Anspruch 5, **dadurch gekennzeichnet, dass** zu Beginn des Aufbereitungsprozesses ein Typ des aufzubereitenden Endoskops (20) bestimmt wird, und dass für den entsprechenden Endoskoptyp die zulässigen Werte für die Änderung der Durchflussrate über die Zeit aus der Datenbank (72) gelesen werden.

## Claims

1. Method for reprocessing an endoscope (20) having at least one channel in a reprocessing device (1), having the steps:
- Introducing the endoscope (20) into the reprocessing device (1),
- Connecting at least one channel of the endoscope (20) to a flushing system of the reprocessing device (1),
- Flushing the at least one channel of the endoscope (1) with a flushing liquid,
wherein a flow rate of the flushing liquid through the at least one channel is measured to detect a complete or partial blockage of the at least one channel or an incomplete connection of the at least one channel to the flushing system,
and the flow rate is measured a plurality of times over a predetermined period of time, **characterized in that** the change in flow rate over time is used to detect blockage of the at least one channel or incomplete connection of the at least one channel to the flushing system.

2. Method according to claim 1, wherein for reprocessing the endoscope (20) a plurality of flushing cycles are performed, **characterized in that** the flow rate is measured over a plurality of predetermined time periods each coinciding with one of the flushing cycles, and that the change in flow rate over time is evaluated separately for each predetermined time period.

3. Method according to claim 1 or 2, wherein the flushing system is a circulating flushing system (60) which is filled with flushing liquid at the beginning of a flushing cycle and is emptied of the flushing liquid after completion of a flushing cycle, **characterized in that** the predetermined period of time is selected such that it at least partially coincides with the filling of the flushing system.

4. Method according to any one of claims 1 to 3, **characterized in that** at least 3, preferably at least 5, particularly preferably at least 7 measured values for the flow rate are evaluated for determining the change in the flow rate over time.

5. Method according to any one of claims 1 to 4, **characterized in that** a predetermined time period is between 6 seconds and 30 seconds, preferably between 8 seconds and 20 seconds, particularly preferably between 10 seconds and 15 seconds.

6. Method according to any one of claims 1 to 5, **characterized in that** the determination of the change in flow rate over time is carried out by means of linear regression.

7. Method according to any one of the preceding claims, **characterized in that** permissible values for the change in flow rate over time are read from a database (72) at the start of the treatment process.

8. Method according to claim 5, **characterized in that** at the beginning of the reprocessing process a type of endoscope (20) to be reprocessed is determined, and that for the corresponding endoscope type the permissible values for the change in flow rate over time are read from the database (72).

## Revendications

1. Procédé de retraitement d'un endoscope (20) comprenant au moins un canal dans un appareil de retraitement (1), comprenant les étapes :
- Introduction de l'endoscope (20) dans l'appareil de retraitement (1),
- Raccordement d'au moins un canal de l'endoscope (20) à un système de rinçage de l'appareil de traitement (1),
- Rinçage d'au moins un canal de l'endoscope (1) avec un liquide de rinçage,
dans lequel un débit du liquide de rinçage à travers le au moins un canal est mesuré afin de détecter un blocage complet ou partiel du au moins un canal ou un raccordement incomplet du au moins un canal avec le système de rinçage,
et le débit est mesuré plusieurs fois sur une période de temps prédéterminée, **caractérisé en ce que** la variation du débit dans le temps est utilisée pour détecter un blocage dudit au moins un canal ou un raccordement incomplet dudit au moins un canal avec le système de rinçage.

2. Procédé selon la revendication 1, dans lequel plusieurs passages de rinçage sont effectués pour traiter l'endoscope (20), **caractérisé en ce que** le débit est mesuré sur plusieurs périodes de temps prédéterminées qui coïncident chacune avec l'un des passages de rinçage, et **en ce que** la variation du débit dans le temps est évaluée séparément pour chaque période de temps prédéterminée.

3. Procédé selon la revendication 1 ou 2, dans lequel le système de rinçage est un système de rinçage à circulation (60) qui est rempli de liquide de rinçage au début d'un cycle de rinçage et qui est vidé du liquide de rinçage à la fin d'un cycle de rinçage, **caractérisé en ce que** la période prédéterminée est choisie de telle sorte qu'elle coïncide au moins partiellement avec le remplissage du système de rinçage.

4. Procédé selon l'une des revendications 1 à 3, **caractérisé en ce que**, pour déterminer la variation du débit en fonction du temps, on évalue au moins 3, de préférence au moins 5, de manière particulièrement préférée au moins 7 valeurs de mesure du débit.

5. Procédé selon l'une des revendications 1 à 4, **caractérisé en ce qu'**une période de temps prédéterminée est comprise entre 6 secondes et 30 secondes, de préférence entre 8 secondes et 20 secondes, de manière particulièrement préférée entre 10 secondes et 15 secondes.

6. Procédé selon l'une des revendications 1 à 5, **caractérisé en ce que** la détermination de la variation du débit en fonction du temps est effectuée au moyen d'une régression linéaire.

7. Procédé selon l'une des revendications précédentes, **caractérisé en ce qu'**au début du processus de retraitement, des valeurs admissibles pour la variation du débit en fonction du temps sont lues dans une base de données (72).

8. Procédé selon la revendication 5, **caractérisé en ce qu'**au début du processus de retraitement, on détermine un type d'endoscope (20) à retraiter, et **en ce que**, pour le type d'endoscope correspondant, des valeurs admissibles pour la variation du débit en fonction du temps sont lues dans la base de données (72).
